# EUROPÄISCHE PATENTSCHRIFT

(11) **EP 1 904 313 B1**
(45) Veröffentlichungstag und Bekanntmachung des Hinweises auf die Patenterteilung: **11.09.2013**
(21) Anmeldenummer: 06754666.3
(22) Anmeldetag: 04.07.2006
(51) Int. Cl.: B43K 29/08, B43K 5/04, G06F 3/0354, A61B 5/11

(54) **FLUIDISCHER STIFT ZUR ERFASSUNG NEUROMOTORISCHER DATEN**
FLUIDIC STICK FOR DETECTING NEUROMOTOR DATA
CRAYON FLUIDIQUE DESTINE A LA DETECTION DE DONNEES NEUROMOTRICES

(30) Priorität: 04.07.2005 DE 102005031432
(43) Veröffentlichungstag der Anmeldung: 02.04.2008
(73) Patentinhaber: Fachhochschule Regensburg, 93049 Regensburg (DE)
(72) Erfinder: KEMPF, Jürgen, 93093 Donaustauf (DE); HOOK, Christian, 93161 Sinzing (DE)
(74) Vertreter: Isarpatent
(86) Internationale Anmeldenummer: PCT/EP2006/006514
(87) Internationale Veröffentlichungsnummer: WO 2007/003417

(56) Entgegenhaltungen:
- EP-A- 0 256 615
- EP-A- 0 349 443
- AT-B- 397 944
- CH-A- 275 827
- DE-A- 2 433 101
- DE-A- 2 442 525
- DE-A- 4 328 312
- DE-A- 10 054 597
- DE-C- 458 683
- US-A- 3 986 403

## Beschreibung

Die Erfindung betrifft einen vielseitig einsetzbaren fluidischen Stift zur Erfassung neuromotorischer und biometrischer Daten, die bei einer handgeführten Bewegung einer Person entstehen.

Neuromotorische Krankheiten werden hauptsächlich durch altersbedingte Krankheiten wie Schlaganfall, Alzheimer, Tremor sowie durch Drogenkonsum wie Alkohol, Nikotin und harte Drogen, durch physischen und mentalen Stress oder durch Unfälle hervorgerufen. Die Erkennung von neuromotorischen Krankheiten und Schreibproblemen sowie die pharmakologische Untersuchung von Wirkungen und Nebenwirkungen von Medikationen, die sich auf das neuromotorische Verhalten einer Person auswirken, gewinnen daher ständig an Bedeutung. Untersuchungen handgeführter Bewegungen beim Schreiben und zeichnen sowie beim Nachfahren von bewegten Objekten und insbesondere die 3D-Erfassung von Gestiken liefern wertvolle Informationen über das neuromotorische Verhalten von untersuchten Personen. Daher sind computerunterstützte Eingabesysteme, die Aufschluss über neuromotorische Störungen, Defizite und Krankheitsverläufe eines Menschen liefern, und die neuromotorischen Merkmale einer handgeführten Bewegung einer Person erfassen von großer Wichtigkeit.

Die DE 24 33 101 beschreibt eine Anordnung von Dehnungsmessstreifen an Tintenfedern von Füllhaltern. Eine Messanordnung dient zur Überprüfung von Tintenfedern für Füllfederhalter mithilfe von Dehnungsmessstreifen.

Die US 3986403 beschreibt ein Schreibinstrument mit einer Fluidkammer zur Aufnahme einer Schreibflüssigkeit. Die Schreibflüssigkeit ist in einem Reservoir enthalten, das durch ein Gehäuse umschlossen wird. Eine beim Schreiben auftretende Druck- oder Zugspannung wird aufgrund einer Distorsion oder Biegung von einem Diaphragma des Gehäuses mithilfe von Dehnungsmessstreifen erfasst.

Heutzutage sind vor allem schreibstiftbasierte Computereingabesysteme bekannt, die sich meist in folgende Klassen einteilen lassen:
Schreibstifte mit Trägheitsmomente gestützter Positions- und Bewegungserkennung bzw. Inertial Navigation Systems (INS), welche als Einzelsysteme relative Ortsänderungen aufzeichnen.
Schreibstifte mit einer extern durchgeführten Ortung bzw. einem External Global Positioning Systems (GPS), welche absolute 2D und 3D-Ortsangaben gegenüber einem vorgegebenen Koordinatensystem liefern. Dies erfordert neben dem entsprechenden Stift zusätzlich externe Einrichtungen und ein vordefiniertes Arbeitsfeld mit dem Koordinatensystem.

Als Beispiel sei der PC-Notestaker als ein Schreibgerät zur Erfassung handgeführter zweidimensionaler Bewegungen mittels Ultraschall-Abstandssensorik für die Text- und Grafikeingabe genannt. Dabei erlaubt ein Ultraschall-Sender im Schreibgerät eine absolute, zweidimensionale Ortsbestimmung, jedoch keine exakte Ortserfassung der Schreibstiftspitze des Schreibgeräts. Darüber hinaus wird die Schreibstiftneigung des Schreibgeräts nicht erfasst. Der PC-Notestaker weist darüber hinaus den Nachteil auf, dass die beim Schreiben auftretende Druckdynamik nicht erfasst wird.

Die verschiedenen Schreibsysteme für computerunterstützte Erfassung handgeführter Bewegungen erfüllen auch nicht in ausreichendem Maße die Anforderungen für einen Einsatz als medizinisches Diagnose- und Therapiesystem, als Biometriesystem oder als Mittel zur Handschriftenerkennung wegen ihrer Ungenauigkeit. So werden beim Stand der Technik die biometrischen und neuromotorischen Informationen nicht umfassend genug und/oder zu ungenau erfasst. Die technischen Realisierungen sind ferner zu aufwändig oder zu kostenintensiv. Im medizinischen und pharmakologischen Bereich ist die schreibstiftbasierte Eingabetechnik bisher nur auf die Verwendung von verhältnismäßig sperrigen Graphiktabletts beschränkt.

Keines der bekannten Eingabesysteme erlaubt die Erfassung neuromotorischer Daten einer handgeführten Schreibbewegung im Raum, wie es bei medizinischen Anwendungen zu Therapie- oder Diagnosezwecken erwünscht ist. Die Verfahren nach dem Stand der Technik erfordern zudem meist spezielle, ebene Schreibunterlagen, um ausschließlich in 2D Bewegungen zu erfassen.

Es ist daher die Aufgabe der vorliegenden Erfindung, ein handliches Instrument zu schaffen, das die Erfassung neuromotorischer Daten einer handgeführten Schreib- oder Zeichenbewegung oder Gestik einer Person, ermöglicht.

Diese Aufgabe wird erfindungsgemäß durch einen fluidischen Stift zur Erfassung neuromotorischer Daten einer handgeführten Bewegung mit in den Patentanspruch 1 angegebenen Merkmalen gelöst.

Ein Vorteil des erfindungsgemäßen fluidischen Stifts besteht darin, dass die handgeführte Bewegung im Raum, d. h. dreidimensional, oder auf einer Schreibunterlage, d. h. zweidimensional, durchgeführt werden kann. Der erfindungsgemäße fluidische Stift zur Erfassung neuromotorischer Daten benötigt somit nicht notwendigerweise eine Schreibunterlage.

Ein weiterer Vorteil des erfindungsgemäßen fluidischen Stifts besteht darin, dass die sensorische Erfassung des Flüssigkeitsdrucks kaum Energie verbraucht.

Der erfindungsgemäße fluidische Stift ist aufgrund der fluidischen Sensorik handlich und klein und kann beliebig in der Hand bei der Erfassung der neuromotorischen Daten gedreht werden, d. h. die sensorische Erfassung der biometrischen Daten ist rotationssymmetrisch zur Längsachse des Stiftes. Eine zu testende Person kann den fluidischen Stift wie einen gewöhnlichen Schreibstift aufgreifen und beliebige Bewegungen im Raum oder auf einer Schreibunterlage durchführen, ohne dass die einzelnen Finger eine spezielle Position an dem Schreibstiftgehäuse einnehmen müssen.

Die fluidische Sensorik des erfindungsgemäßen Stifts kann auf kleinstem Raum eingebaut werden und arbeitet rotationssymmetrisch.

Der erfindungsgemäße fluidische Stift erfasst mit hoher Sensibilität und Selektivität die neuromotorischen Eigenschaften der Feinmotorik von Fingern, Hand, Arm und Schulter einer Person.

Bei einer bevorzugten Ausführungsform des erfindungsgemäßen fluidischen Stiftes weist die Fluidkammer einen ersten Fluidkammerabschnitt auf, der spiral- oder ringförmig ist.

Bei einer bevorzugten Ausführungsform des erfindungsgemäßen fluidischen Stiftes weist die Fluidkammer einen zweiten Fluidkammerabschnitt auf, der kanalförmig ist und in Längsrichtung des Stiftes angeordnet ist.

Bei dem in der Fluidkammer enthaltenen Fluid handelt es sich vorzugsweise um eine elektrisch leitende, elektrisch nicht leitende oder magneto-rheologische Flüssigkeit.

Die in dem erfindungsgemäßen fluidischen Stift vorgesehenen Drucksensoren erfassen vorzugsweise den Flüssigkeitsdruck innerhalb der Fluidkammer kapazitiv, elektroresistiv oder induktiv.

Die elastische Wandung besteht vorzugsweise aus einem elastischen Schlauch.

Bei einer Ausführungsform des erfindungsgemäßen fluidischen Stiftes wird der ringförmige erste Fluidkammerabschnitt durch eine ringförmige Nut in einem Stiftkörper des Stiftes gebildet, die mit einer elastischen Hülse überzogen ist.

Diese elastische Hülse weist bei einer bevorzugten Ausführungsform auf der Innen- oder Außenseite eine elastische Piezo-Schicht auf.

Die elastische Hülse überzieht vorzugsweise den ringförmigen Fluidkammerabschnitt und zumindest teilweise den zylinderförmigen Stiftkörper.

Bei einer bevorzugten Ausführungsform des erfindungsgemäßen fluidischen Stiftes weist der spiralförmige erste Fluidkammerabschnitt mehrere Windungen auf, welche auf mindestens einer elastischen Piezo-Folie aufliegen, die auf einer elastischen Unterlage aufgebracht ist.

Dabei ist die elastische Piezo-Folie vorzugsweise streifenförmig und in Längsrichtung des Stiftes angeordnet oder optional mit einem elastischem Medium beschichtet, gewickelt oder gefaltet.

Bei einer bevorzugten Ausführungsform des erfindungsgemäßen fluidischen Stiftes ist für jeden Finger, der den Stift bei der handgeführten Bewegung greift, eine zugehörige Piezo-Folie vorgesehen.

Der erfindungsgemäße fluidische Stift weist vorzugsweise eine auf eine Unterlage aufsetzbare Stiftmine auf.

Bei einer bevorzugten Ausführungsform des erfindungsgemäßen fluidischen Stiftes weist die Stiftmine eine Stiftspitze auf. Die Position der Spitze und die Neigung der Mine werden mittels akustischer Abstandsmessung bestimmt.

Hierzu weist der erfindungsgemäße fluidische Stift vorzugsweise an der Stiftspitze und an einem gegenüberliegenden distalen Ende des Stiftes jeweils einen Ultraschall-Wandler auf.

Bei einer ersten Ausführungsform sind die an dem fluidischen Stift angebrachten Ultraschall-Wandler Ultraschall-Empfänger zum Empfangen eines Ultraschall-Signals.

Bei einer alternativen Ausführungsform des erfindungsgemäßen fluidischen Stiftes sind die an dem fluidischen Stift angebrachten Ultraschall-Wandler Ultraschall-Sender zum Senden eines Ultraschall-Signals.

Bei einer bevorzugten Ausführungsform des erfindungsgemäßen fluidischen Stiftes wird ein auf die Spitze der Stiftmine aufgebrachte Schreibdruck durch Magnetfeldsensoren erfasst.

Diese Magnetfeldsensoren sind vorzugsweise entweder GMR-Sensoren oder Hall-Sensoren.

Bei einer bevorzugten Ausführungsform des erfindungsgemäßen fluidischen Stiftes ist an dem der Stiftmine entgegengesetzten distalen Ende ein Permanentmagnet vorgesehen, der ein inhomogenes Magnetfeld erzeugt, dessen Änderungen bei Bewegung der Stiftmine durch einen Magnetfeldsensor erfasst wird.

Bei einer bevorzugten Ausführungsform des erfindungsgemäßen fluidischen Stiftes ist der Magnetfeldsensor zwischen den Permanentmagneten und einem zylinderförmigen ferromagnetischen Körper, beispielsweise einem Eisenring, angeordnet.

Im Weiteren werden bevorzugte Ausführungsformen des erfindungsgemäßen fluidischen Stiftes zur Erfassung neuromotorischer Daten unter Bezugnahme auf die beigefügten Figuren gezeigt.

Es zeigen:
Figur 1: eine erste Ausführungsform des erfindungsgemäßen fluidischen Stiftes zur Erfassung neuromotorischer Daten einer handgeführten Bewegung;
Figur 2: eine Querschnittansicht der ersten Ausführungsform des erfindungsgemäßen fluidischen Stiftes zur Erfassung neuromotorischer Daten einer handgeführten Bewegung gemäß der Erfindung;
Figur 3: eine zweite Ausführungsform des erfindungsgemäßen fluidischen Stiftes zur Erfassung neuromotorischer Daten einer handgeführten Bewegung mit einer Piezofolie;
Figuren 4a, 4b: Alternative Ausführungsformen des erfindungsgemäßen fluidischen Stiftes zur Erfassung neuromotorischer Daten einer handgeführten Bewegung mit einer Piezofolie;
Figur 5: eine erste Ausführungsform eines innerhalb des erfindungsgemäßen fluidischen Stiftes eingesetzten Drucksensors;
Figur 6: eine zweite Ausführungsform eines innerhalb des erfindungsgemäßen fluidischen Stifts eingesetzten Drucksensors;
Figur 7: eine Querschnittansicht einer ersten magnetischen Drucksensorik zur Erfassung eines auf eine Schreibstiftmine des fluidischen Stiftes ausgeübten Schreibdruckes;
Figur 8: eine Querschnittansicht einer alternativen magnetischen Drucksensorik zur Erfassung eines auf eine Schreibstiftmine des fluidischen Stiftes ausgeübten Schreibdruckes;
Figur 9: eine weitere Ausführungsform des erfindungsgemäßen fluidischen Stiftes zur Erfassung neuromotorischer Daten mit mehreren zusätzlichen sensorischen Erfassungseinheiten;
Figur 10: ein fluidisches Stiftmodul als Stand Alone-System;
Figur 11: einen erfindungsgemäßen fluidischen Stift in Verbindung mit einem Smart Display;
Figur 12: den erfindungsgemäßen fluidischen Stift in Verbindung mit einem Notebook.

Im weiteren werden bevorzugte Ausführungsformen des erfindungsgemäßen fluidischen Stiftes zur Erläuterung erfindungswesentlicher Merkmale unter Bezugnahme auf die beigefügten Figuren beschrieben.

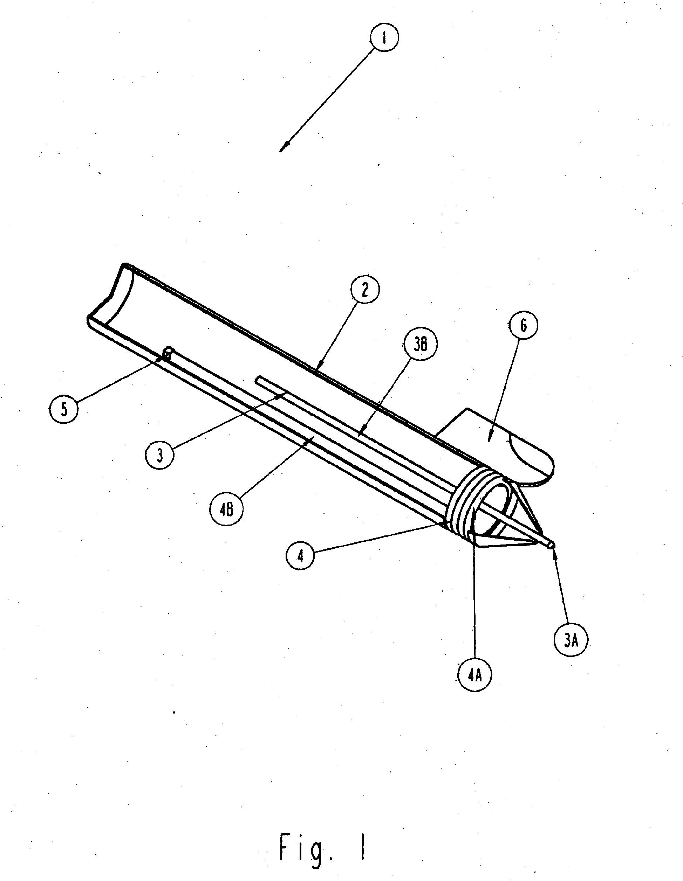

Wie man aus Figur 1 erkennen kann, weist der erfindungsgemäße fluidische Stift 1 ein Stiftgehäuse 2 auf, in dem eine Stiftmine 3 gelagert ist. Die Stiftmine 3 weist eine Stiftspitze 3A und ein Stiftende 3B auf. In dem fluidischen Stift 1 ist eine Fluidkammer 4 vorgesehen, die aus zwei Fluidkammerabschnitten besteht. Bei der in Figur 1 dargestellten ersten Ausführungsform ist der erste Fluidkammerabschnitt 4A helix- bzw. spiralförmig und weist mehrere Windungen bzw. Wicklungen auf. Der zweite Fluidkammerabschnitt 4B der Fluidkammer ist kanalförmig und verläuft in Längsrichtung des fluidischen Stiftes 1, beispielsweise parallel zu der Stiftmine 3. Am Ende des kanalförmigen Fluidkammerabschnitts 4B ist ein Drucksensor 5 vorgesehen.

Der Drucksensor 5 erfasst den Flüssigkeitsdruck P innerhalb der Fluidkammer 4 kapazitiv, elektroresistiv oder induktiv.

Die in der Fluidkammer 4 enthaltene Flüssigkeit kann entweder elektrisch leitend, nicht leitend sein.

Bei einer alternativen Ausführungsform ist das in der Fluidkammer 4 enthaltene Fluid eine magnetische Flüssigkeit, sodass der Drucksensor 5 die Flüssigkeitsdruckschwankungen innerhalb der Fluidkammer 4 induktiv erfassen kann.

Bei alternativen Ausführungsformen handelt sich bei dem Drucksensor um einen mikromechanischen Drucksensor, wobei der Druck der Fluidkammer piezoresistiv erfasst wird.

Der Flüssigkeitsdruck P innerhalb der Fluidkammer 4 entspricht der bei einer handgeführten Bewegung auftretenden Kraft. Dabei kann es sich um eine beliebige handgeführte Bewegung im Raum, d. h. dreidimensional, oder auf einer Schreibunterlage, d. h. zweidimensional, handeln. Die bei der handgeführten Bewegung auftretenden Kräfte umfassen einerseits die bei einer Beschleunigung a auftretenden Trägheitskräfte und andererseits die durch die Greiffinger 6 aufgebrachten Fingerdruckkräfte, die durch Muskelkontraktion entstehen. Üblicherweise wird das Schreibstiftgehäuse 2 des fluidischen Schreibstiftes 1 mit drei Fingern gegriffen, nämlich mit dem Daumen, dem Zeigefinger und dem Mittelfinger. Wie in Figur 1 erkennbar, führt das Drücken der Finger 6 auf den spiralförmigen ersten Fluidkammerabschnitt 4A der Fluidkammer 4 zu einer Veränderung des Flüssigkeitsdrucks P innerhalb der Fluidkammer 4. Zur Erfassung des Greifdrucks ist der spiralförmige Schlauch der Fluidkammer 4 um das Schreibstiftgehäuse 2 gewickelt und die Finger drücken auf den gewickelten Schlauch.

Bei der in Figur 1 gezeigten Ausführungsform ist das Schlauchende mit dem fluidischen Drucksensor 5 abgeschlossen. Bei einer alternativen Ausführungsform sind beide Enden des Schlauches bzw. der Fluidkammer 4 mit einem fluidischen Drucksensor 5 abgeschlossen.

Die in Figur 1 dargestellte Ausführungsform zeichnet sich dadurch aus, dass die Fluidkammer 4 technisch einfach zu realisieren ist und darüber hinaus kompakt, wenig störanfällig und äußerst flexibel in das Schreibstiftgehäuse 2 integrierbar ist. Die Empfindlichkeit, die Dynamik, der Messbereich und die Messgeschwindigkeit lassen sich über die Geometrie, d. h. über die Dicke, den Querschnitt und die Anzahl der Windungen und das eingesetzte Material der Schlauchwandung einstellen. Die Fluidkammer 4 weist eine Wandung auf, die erfindungsgemäß aus einem elastischen Material besteht. In der Fluidkammer 4 befindet sich entweder eine Flüssigkeit oder ein Gel. Die Fluidkammer 4 erfasst den Fingerdruck und überträgt diesen Fingerdruck bzw. die Greifkraft über das Fluid an den Drucksensor 5. Die rein fluidmechanische Erfassung und Übertragung des Fingerdrucks erfolgt ohne elektrische Wandlung und ohne elektrische Signalübertragung des erfassten Druckes zum Sensor 5.

Dies hat den Vorteil, dass die Übertragung zum Drucksensor keine elektrische Energie verbraucht. Ein weiterer Vorteil besteht darin, dass die fluidische Sensorik äußerst EMVverträglich ist.

Ein weiterer Vorteil der fluidischen Sensorik besteht darin, dass die auftretenden Fingerdruckkräfte rotationssymmetrisch zur Längsachse des Schreibstiftes 1 erfasst werden. Hierdurch ist es möglich, dass die zu untersuchende Person den Schreibstift 1 wie einen gewöhnlichen Stift verwenden kann, ohne auf eine bestimmte Position der Finger 6 auf dem Schreibstiftgehäuse 2 achten zu müssen.

Der Drucksensor 5 erfasst den Flüssigkeitsdruck P und wandelt ihn in ein elektrisches Signal für die weitere Datenverarbeitung durch eine Datenverarbeitungseinheit um. Die Datenverarbeitungseinheit ist bei einer ersten Ausführungsform innerhalb des Schreibstiftgehäuses 2 integriert. Bei einer alternativen Ausführungsform befindet sich die Datenverarbeitungseinheit außerhalb des Schreibstiftgehäuses 2. Die erzeugten elektrischen Datensignale werden dann über eine Interface-Schaltung entweder über eine drahtgebundene Leitung oder drahtlos an die Datenverarbeitungseinheit von dem fluidischen Stift 1 übertragen. Die Daten werden bei bestimmten Anwendungen vorzugsweise verschlüsselt übertragen. Der verdrahtet oder drahtlos mit einem Computer verbundene fluidische Stift 1 gibt die elektrischen Signale an die Datenverarbeitungseinheit bzw. den Computer ab, der zur medizinischen Diagnose und Therapie die neuromotorischen Daten bzw. Datensignalen aus geschriebenen und gezeichneten Vorgaben, wie standardisierte Symbole, Figuren, Wörter oder Texte sowie bewegten Objekten gewinnt. Dabei werden die Vorgaben frei geschrieben, gezeichnet, überschrieben oder überzeichnet. Alternativ fährt die zu untersuchende Person bewegte Objekte auf einem Display nach.

Bei einer alternativen Anwendung des erfindungsgemäßen fluidischen Stiftes 1, wie er in Figur 1 dargestellt ist, werden biometrische Daten aus handgeschriebenen Gegenständen wie Zeichen, Wörter, Texte, Signaturen oder Pins gewonnen. Die dabei gemessenen Sensorzeitsignale der handgeführten Bewegung sind derart detailliert, dass bereits kurze geschriebene Schreibsequenzen genügen, um eine biometrische Klassifizierung und Identifizierung einer Person zu ermöglichen oder um bestimmte individuelle neuromotorische Verhaltensänderungen zu registrieren.

Mittels einer Datenverarbeitungssoftware werden aus den fluidischen Sensordaten des Stiftes 1 neuromotorische Verhaltensmerkmale gewonnen, die eine detaillierte, quantitative und schnelle Analyse sowie Interpretationen von Einflussfaktoren wie genetische Veranlagung, erworbene Verhaltensmuster sowie Krankheiten, Medikamente, Drogen und Stress auf das neuromotorische Verhalten der Finger 6, des Handgelenks und des Arms ermöglichen. Weiter lassen sich mit den beidseitig erfassten biometrischen Verhaltensmerkmalen Personenidentifikations-und Zugangskontrollen durchführen. Der vertraute Umgang mit einem Schreibstift 1 führt zu einer hohen Akzeptanz beim Benutzer und zu einer einfachen Bedienweise.

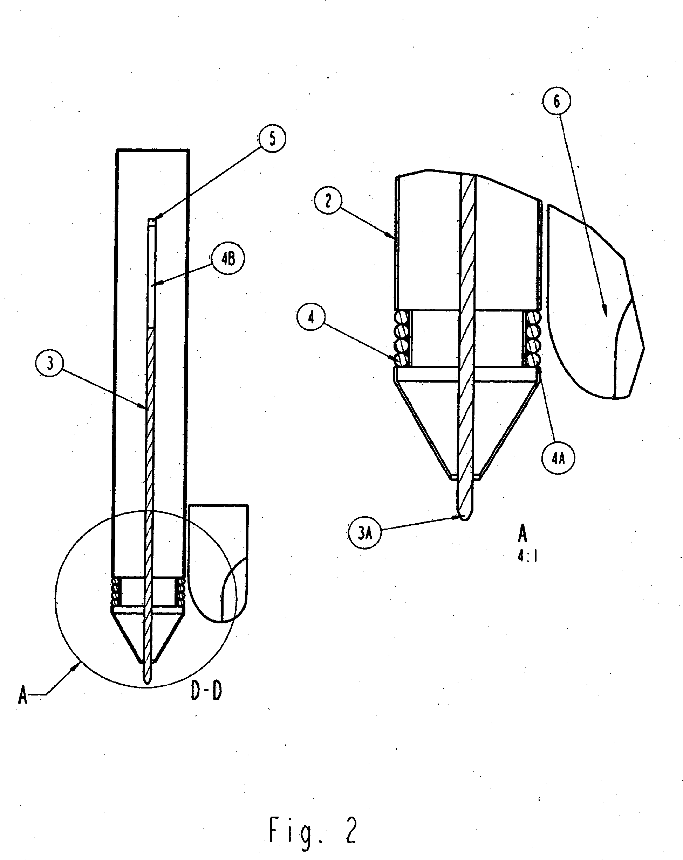

Figur 2 zeigt eine Querschnittansicht durch den erfindungsgemäßen fluidischen Stift 1 gemäß der ersten Ausführungsform. Der auf den elastischen Schlauch 4 aufgebrachte Greifdruck der Finger 6 wird auf das in der Fluidkammer 4 enthaltene Fluid bzw. ein darin enthaltene Gel oder eine Flüssigkeit übertragen. In der Flüssigkeit breitet sich der allseitig wirkende Flüssigkeitsdruck P mit Schallgeschwindigkeit aus. Beispielsweise liegt die Schallgeschwindigkeit bei Wasser und bei einer Temperatur von 20°C bei 1384 m pro Sekunde. Der Drucksensor 5 misst den Hydrodruck, der im Allgemeinen im Niederfrequenzbereich unter 100 Hz liegt. Wie man aus Figur 2 erkennen kann, ist der helix-förmige bzw. spiralförmige Fluidkammerabschnitt 4A der Fluidkammer 4 vorzugsweise in einer ringförmigen Nut des Stiftkörpers 2 gewickelt. Bei der in Figur 2 dargestellten Ausführungsform weist die Fluidkammer 4 beispielsweise vier Wicklungen auf.

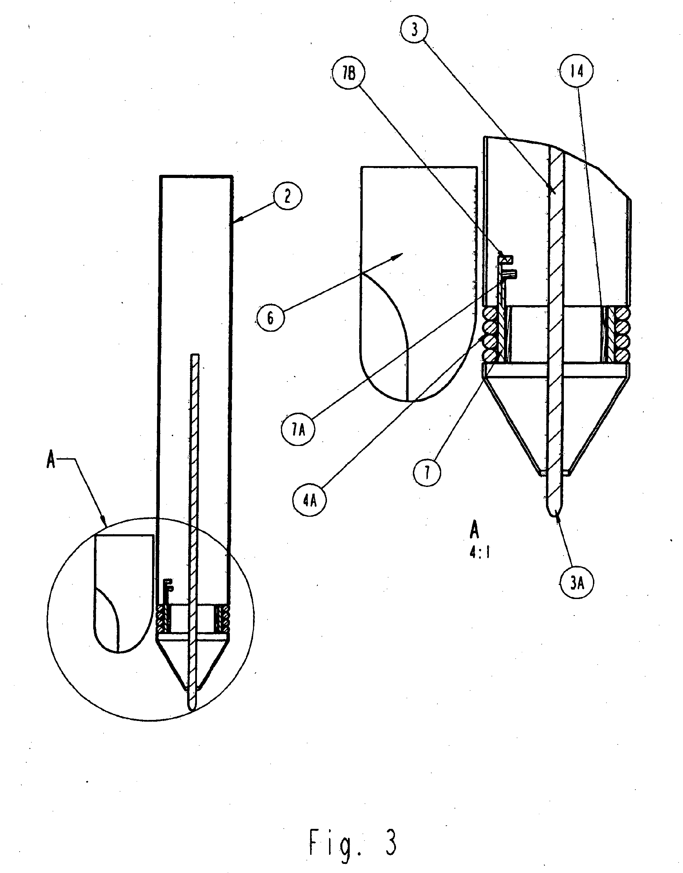

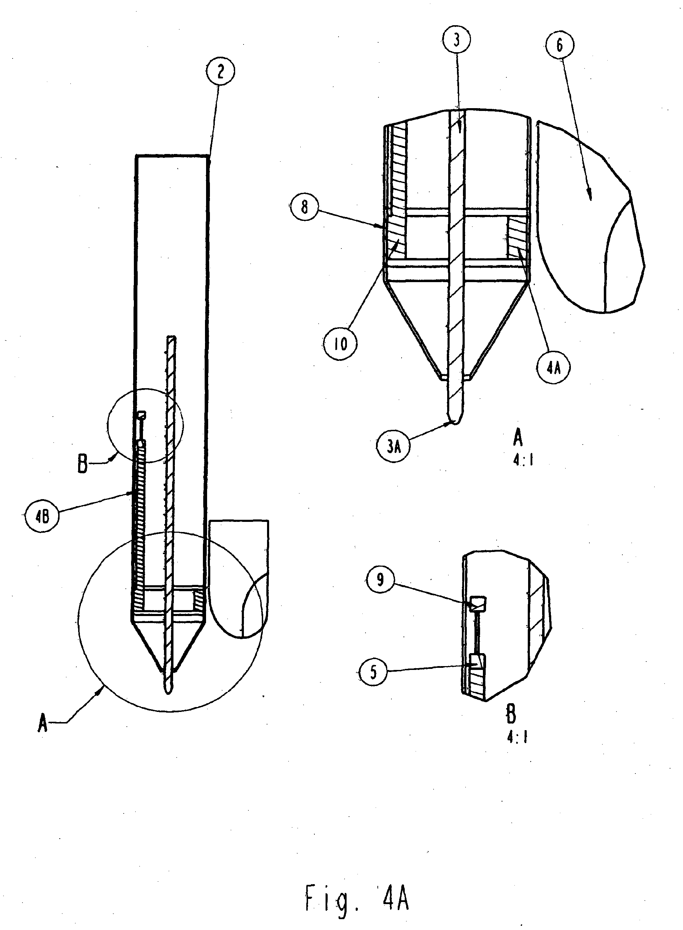

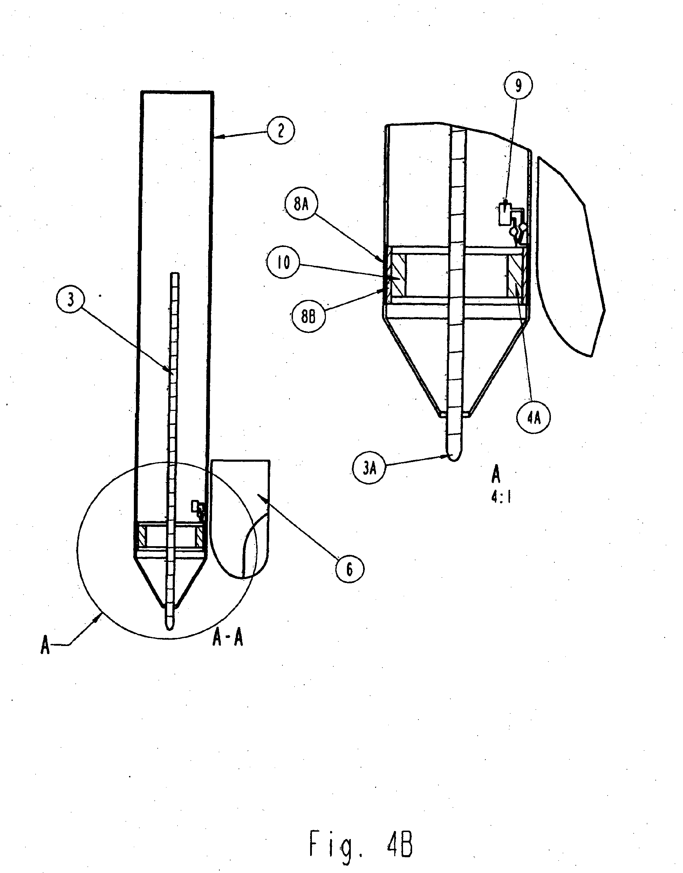

Figur 3 zeigt eine weitere bevorzugte Ausführungsform des erfindungsgemäßen fluidischen Stiftes 1. Bei dieser Ausführungsform ist neben der fluidischen Sensorik zusätzlich eine elastische Piezo-Folie 7 vorgesehen. Die Piezo-Folie 7 ist unterhalb der spiralförmigen Wicklungen auf einer elastischen Schicht aufgebracht. Die Piezo-Folie.7 weist zwei Signalanschlüsse 7A, 7B zur Übertragung der elektrischen Sensorsignale auf. Der Greifdruck der Finger 6 deformiert hierbei nicht nur den Schlauch 4 sondern auch die Piezo-Folie 7, welche auf dem elastischen Material gelagert ist. Dabei entsteht an den Anschlüssen 7A, 7B ein piezoelektrisches Spannungssignal, welches dem aufgebrachten Fingerdruck entspricht. Figuren 4a, 4b zeigen weitere bevorzugte Ausführungsformen des erfindungsgemäßen fluidischen Stiftes 1 zur Erfassung neuromotorischer Daten bei einer handgeführten Bewegung. Figur 4a zeigt eine ringförmige Nut, die mit einer elastischen Hülse 8 überzogen ist und mit einem Fluid 10 gefüllt ist. Die gefüllte Nut bildet den ersten Fluidkammerabschnitt 4A.

Bei der in Figur 4b dargestellten Ausführungsform des fluidischen Stiftes 1 wird der ringförmige erste Fluidkammerabschnitt 4A durch eine ringförmige Nut in dem aus Metall oder Kunststoff bestehenden Stiftkörper 2 des fluidischen Stiftes 1 gebildet, die ebenfalls mit einer elastischen Hülse 8 überzogen ist. Diese elastische Hülse 8 besteht aus einer ersten elastischen Schicht 8A und aus mindestens einer zweiten elastischen Piezo-Schicht 8B, die vorzugsweise auf die erste Schicht 8A aufgebracht ist. Vorzugsweise sind drei radial beabstandete Piezo-Schichten 8B für die drei Greiffinger 6 auf der Innenseite der elastischen Schicht 8A aufgebracht.

Bei den in Figur 4 dargestellten Ausführungsformen ist eine Datenverarbeitungseinheit 9 innerhalb des fluidischen Stiftes 1 integriert die Sensorsignale von dem Drucksensor 5 und/oder Sensorsignale von der piezoelektrischen Schicht 8B der Hülse 8 empfängt. Die elastische Hülse 8 überzieht den ringförmigen Fluidkammerabschnitt 4A vollständig und zumindest teilweise den zylinderförmigen Stiftkörper 2, wie man aus Figur 4b erkennen kann. Das hat zur Folge, dass die Piezo-Schicht 8B der elastischen Hülse 8 gemäß Figur 4b sowohl die Fingerkräfte, die auf den ersten Fluidkammerabschnitt 4A ausgeübt werden, als auch Körperschwingungen des Stiftkörpers 2 elektrisch erfasst. Schwingungen der Stiftmine 3 werden auf den Stiftkörper 2 übertragen und werden im Bereich des direkten Kontakts der Hülse 2 mit dem Stiftkörper 2 durch die Piezo-Schicht 8B erfasst und als Sensorsignale an die Datenverarbeitungseinheit 9 angelegt. Die Piezo-Schicht 8B erfasst somit gleichzeitig die relativ hochfrequenten Schwingungen der Stiftmine 3 und die relativ niederfrequenten Schwingungen der auftretenden Fingerkräfte. Diese Signalanteile lassen sich mit elektrischen Filtern trennen.

Die in den Ausführungsformen gemäß der Figuren 3, 4 verwendeten Piezo-Folien 7 bzw. Piezo-Schichten 8B sind vorzugsweise als Streifen ausgebildet, die in Längsrichtung des Stiftes 1 angeordnet sind. Dabei wird vorzugsweise für jeden Finger 6, d. h. für den Daumen, den Mittelfinger und den Zeigefinger, die den Stift 1 halten, eine zugehörige streifenförmige Piezo-Folie vorgesehen. Die verschiedenen Piezo-Folien erfassen separat die durch den jeweiligen Finger aufgebrachten Fingerdruckkräfte und geben somit zusätzliche Informationen bezüglich der Neuromotorik der verschiedenen Finger ab.

Zur Steigerung der Druckempfindlichkeit werden die Piezofolien mit einem elastichem Material beschichtet und mehrfach gefaltet oder aufgerollt eingebaut.

Die in den Figuren 1, 2, 4 dargestellten verschiedenen Ausführungsformen des erfindungsgemäßen fluidischen Stiftes 1 können beliebige handgeführte Bewegungen ausführen, d. h. im Raum oder auf einer Schreibunterlage. Bei einer Schreibbewegung auf einer Schreibunterlage wird die Stiftminenspitze 3A auf die Unterlage aufgesetzt und wird auf der Schreibunterlage bewegt.

Die Stiftmine 3 gibt bei einer ersten Ausführungsform keine Schreibflüssigkeit ab.

Bei einer zweiten Ausführungsform gibt die Stiftmine 3 eine Schreibflüssigkeit, beispielsweise Tinte, auf die Schreibunterlage ab. Bei der Schreibunterlage kann es sich beispielsweise um Papier handeln. Der Vorteil dieser zweiten Ausführungsform besteht darin, dass dies für die zu untersuchende Person zu einem völlig normalen Schreibvorgang führt, sodass ein natürliches Schreibverhalten hervorgerufen wird, das zur Erfassung neuromotorischer Daten besonders geeignet ist.

Bei einer alternativen Ausführungsform handelt es sich bei der Schreibunterlage um eine kratzfeste Anzeigeoberfläche eines Displays. Bei der Display-Unterlage besteht die Möglichkeit, bewegte Objekte auf einem Monitor mit dem fluidischen Stift 1 nachzufahren, sodass Reaktionsgeschwindigkeit, Konzentration und Aufmerksamkeit der zu untersuchenden Person erfasst werden können.

Bei einer weiteren alternativen Ausführungsform handelt es sich bei der Schreibunterlage um ein Grafiktablett eines PCs.

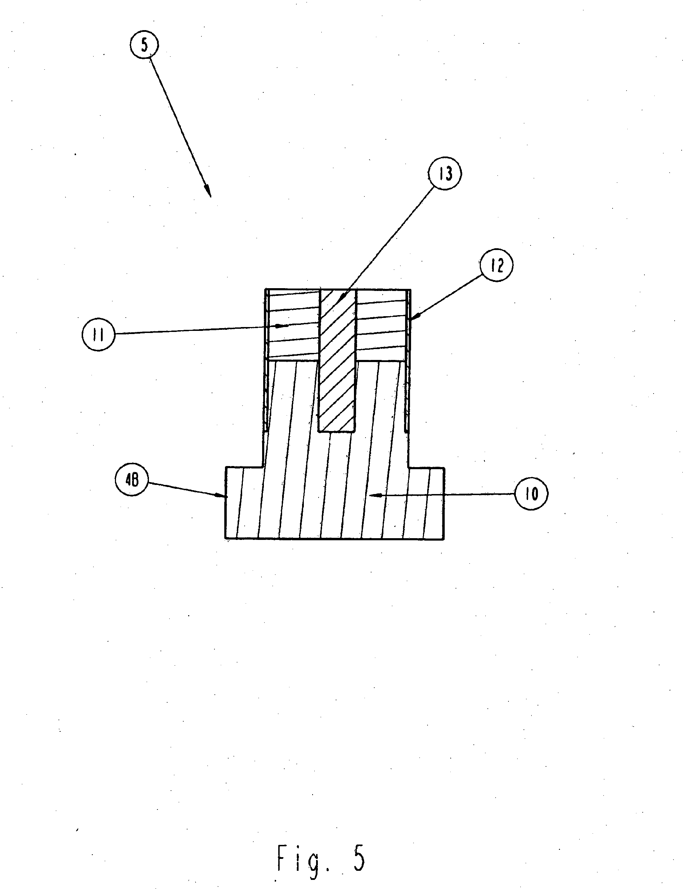

Figur 5 zeigt eine erste Ausführungsform eines bei dem erfindungsgemäßen fluidischen Stiftes eingesetzten Drucksensors 5. Der in Figur 5 dargestellte Drucksensor 5 ist ein kapazitiver oder elektroresistiver Drucksensor, der an dem zweiten Fluidkammerabschnitt 4B angebracht ist. In der Fluidkammer 4 befindet sich ein Fluid 10 und an einem verjüngten Kammerende ein Gas 11. Zur kapazitiven Erfassung der Druckschwankungen sind zwei Metallelektroden 12, 13 vorgesehen. Der Flüssigkeitsdruck wird über die druckabhängige Volumenänderung des Gasraums im Sensorkanal gemessen.

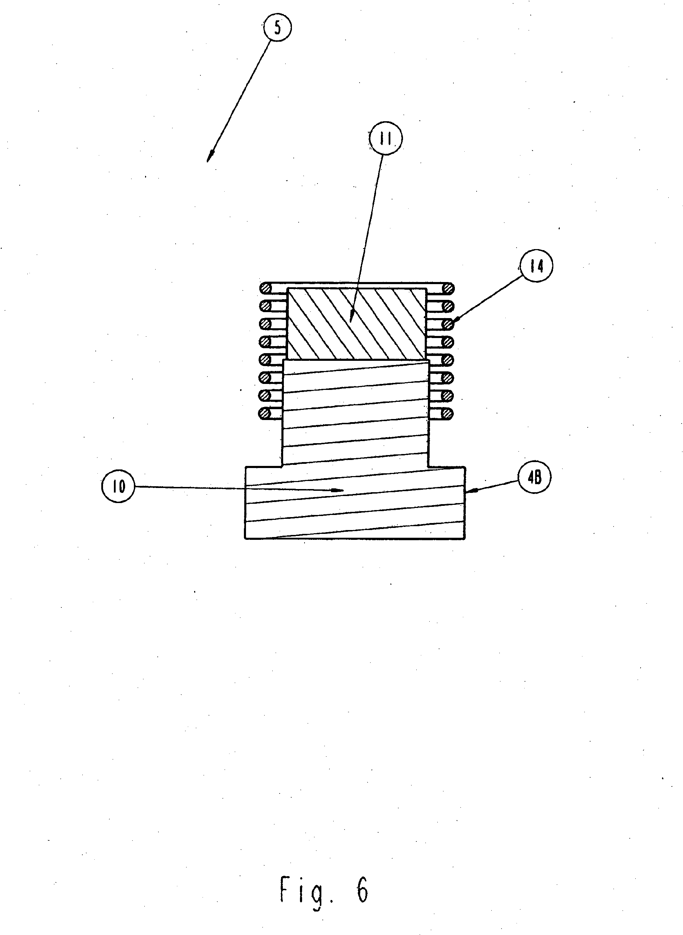

Figur 6 zeigt eine alternative Ausführungsform des in dem erfindungsgemäßen fluidischen Stift 1 eingesetzten Drucksensors 5. Der verjüngte Endabschnitt des zweiten Fluidkammerabschnitts 4B ist mit einer Draht-Spule 14 zur induktiven Erfassung von Druckänderungen versehen, wobei die Flüssigkeit magneto-rheologische Eigenschaften aufweist.

Bei den in Figuren 5, 6 dargestellten Drucksensoren 5 wird eine Druckänderung zur Veränderung des Volumens des Gases 11 eingesetzt und dies führt wiederum zu einer Änderung des elektrischen Widerstands bzw, der Kapazität bzw. der Induktivität. Die Drucksensoren 5 gemäß Figuren 5 und 6 sind einfach und robust und technisch leicht realisierbar. Wegen der rotationssymmetrischen Anordnung des Fluidkanals 4B sind die Signale unabhängig von der Schreibgerätneigung und einem Drehwinkel des Schreibstiftes 1. Ist das Fluid 10 elektrisch leitend, lässt sich die druckabhängige Volumenänderung zwischen Elektroden über Widerstandsänderungen bestimmen. Ist das Fluid nicht elektrisch leitend, ändert die druckabhängige Volumenänderung die Kapazität zwischen den Metallelektroden 12, 13 des in Figur 5 dargestellten Drucksensors 5.

Bei dem in Figur 6 verwendeten Drucksensor 5 ist das Fluid 10 eine magnetisch-rheologische Flüssigkeit, die die Induktivität der Spule 14 bei Druckschwankungen ändert.

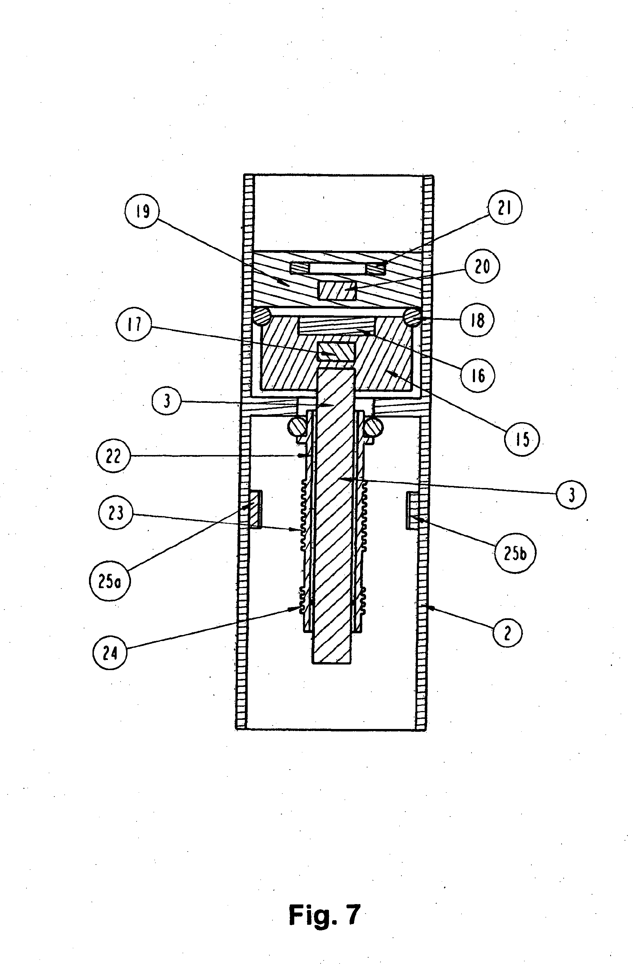

Figur 7 zeigt eine bevorzugte Ausführungsform des erfindungsgemäßen fluidischen Stiftes 1 mit einer magnetischen Drucksensorik zur Erfassung eines auf die Schreibstiftmine 3 ausgeübten Schreibdruckes. Am distalen Ende der Schreibstiftmine 3 ist eine Halterung 15 angebracht, in die ein Permanentmagnet 16 eingelassen ist. Darüber hinaus enthält die Halterung 15 bei der in Figur 7 dargestellten Ausführungsform ein Mikrophon 17. Die Halterung 15 liegt über einen elastischen Gummiring 18 an einer weiteren Halterung 19 an, in die ein magnetischer Feldsensor 20 eingelassen ist. Bei dem magnetischen Feldsensor 20 handelt es sich entweder um einen GMR-Sensor oder bevorzugtermaßen um einen Hall-Sensor. Des Weiteren enthält die Halterung 19 einen ringförmigen ferromagnetischen Körper, beispielsweise einen Eisenring 21.

Die Schreibstiftmine 3 befindet sich in einer Schreibminenhülse 22, die teilweise mit einer magnetischen Schicht 23 und mit einer Piezo-Folie 24 versehen ist. Im Bereich der magnetischen Schicht 23 sind auf der Innenseite des Schreibstiftgehäuses 2 weitere magnetische Feldsensoren 25A, 25B vorgesehen.

Die in Figur 7 dargestellte Sensorik dient der magnetischen Messung des Schreibdruckes längs und quer zur Schreibstiftmine 3. Der Permanentmagnet 16 ist mit dem Ende der frei beweglichen Schreibstiftmine 3 verbunden. Beim Schreiben wird abhängig vom Schreibdruck die Schreibstiftmine 3 mit dem Permanentmagneten 16 relativ zu dem Magnetfeldsensor 20 in xyz-Richtung bewegt. Der mit dem Gehäuse 2 verbundene Magnetfeldsensor 20 befindet sich in einem inhomogenen Magnetfeld, das in Bezug auf die Längsachse der Stiftmine 3 rotationssymmetrisch ist. Der Schreibstift 1 kann daher beim Schreiben beliebig gedreht gehalten werden. Durch den Eisenring 21 hinter dem Magnetfeldsensor 20 wird die Inhomogenität des Magnetfeldes derart gestaltet, dass x-, y- und z-Bewegungen in dem Magnetfeldsensor 20 große Änderungen der magnetischen Felddichte hervorrufen. Bei dem in Figur 7 dargestellten Sensor wird ein Schreibdruck nicht nach xyz-Anteilen aufgelöst, sondern als überlagerte Signale gemessen. Der elastische Gummiring 18 stellt die Stiftmine 3 in die Ruhelage zurück. Über die Elastizität des Materials des Gummirings 18 lässt sich der dynamische Bereich der Schreibdrucksensorik einstellen. Ein Vorteil des in Figur 7 dargestellten Drucksensors besteht darin, dass alle xyz-Schreibdruckbeiträge erfasst werden. Der Drucksensor misst nicht nur die Druckänderungen, sondern auch den Absolutdruck, der beim Schreiben auf die Schreibstiftmine 3 ausgeübt wird. Die Übertragung des auf die Schreibstiftmine 3 ausgeübten Schreibdruckes auf den Sensor erfolgt berührungsfrei.

Zusätzlich erfolgt optional eine Messung des quer zur Schreibstiftmine 3 auftretenden Schreibdrucks mittels der magnetischen Schicht 23 mit den Magnetsensoren 25. Des Weiteren werden die beim Schreiben erzeugten Schwingungen der Stiftmine 3 als Körperschall mittels der Piezo-Folie 24 erfasst. Dazu ist die Piezo-Folie 24 mechanisch eng mit der Hülse 22 verbunden.

Der in Figur 7 dargestellte neuartige Drucksensor dient zur Erfassung einer Relativbewegung zwischen einem ersten Körper, nämlich der Stiftmine 3 und einem zweiten Körper, nämlich dem Stiftgehäuse 2. An dem ersten Körper 3 ist der Permanentmagnet 16 angebracht und an dem zweiten Körper 2 ist der Magnetfeldsensor 20 angebracht. Die Besonderheit des neuartigen Bewegungssensors, wie er in Figur 7 dargestellt ist, besteht darin, dass der Magnetfeldsensor 20 zwischen dem Permanentmagneten 16 und einem an dem zweiten Körper 2 angebrachten ferromagnetischen Ring 21 angeordnet ist. Dieser ferromagnetische Ring 21 besteht beispielsweise aus Eisen. Der Eisenring 21 sorgt dafür, dass sich der Magnetfeldsensor 20 in einem rotationssymmetrischen inhomogenen Magnetfeld befindet und besonders empfindlich auf Relativbewegungen des ersten Körpers 3 zu dem zweiten Körper 2 reagiert. Der neuartige Magnetfeldsensor gemäß Figur 7 erfasst alle in xyz-Richtung auftretenden Relativbewegungen und arbeitet berührungsfrei.

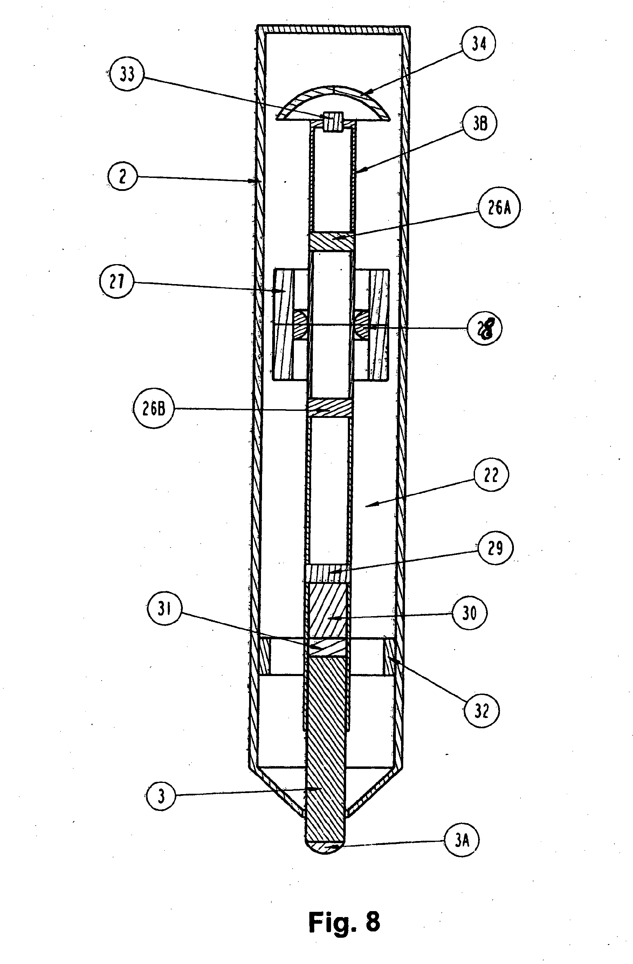

Figur 8 zeigt eine alternative Ausführungsform der magnetischen Drucksensorik innerhalb des erfindungsgemäßen fluidischen Stiftes 1 zur Erfassung des auf die Schreibstiftmine 3 ausgeübten Schreibdruckes. Der Schreibstift 1 enthält innerhalb des Schreibstiftgehäuses 2 magnetische Feldsensoren, insbesondere Hall-Sensoren 26, einen Ringmagneten 27 sowie einen unmagnetischen Führungsring 28. Darüber hinaus ist ein magnetischer Feldsensor, insbesondere ein Hall-Sensor 29, vorgesehen, der über eine elastische Federung 30 an einem Stabmagneten 31 anliegt. Die in der Minenhülse 22 gelagerte Stiftmine 3 ist mittels einer elastischen Abstandshalterung 32 in dem Stiftgehäuse 2 montiert. Das distale Ende 3B der Stiftmine 3 liegt über einer Teflonkugel 33 in einer kappenförmigen Führung 34, ähnlich einem Kugelgelenk in einer Gelenkpfanne.

Bei der in Figur 8 dargestellten Schreibdrucksensorik lassen sich die Druckkräfte längs und quer zur Mine 3 einzeln erfassen. Die Hall-Sensoren 26A, 26B sind an der Minenhülse 22 fest angebracht und bewegen sich über Kippbewegungen der Stiftmine 3 in einem radial symmetrischen inhomogenen Magnetfeld. Das Magnetfeld stammt von dem Ringmagneten 27, der fest mit dem Stiftkörper 2 verbunden ist. Durch die Bewegung ändern sich die Feldflussdichten in beiden Sensoren 26A, 26B. Daraus lässt sich der Schreibdruck quer zur Schreibstiftmine 3 bestimmen. Der Hall-Sensor 29 im Feld des Magneten 31 erfasst die Bewegungen zusammen mit dem Schreibdruck längs zur Schreibstiftmine 3.

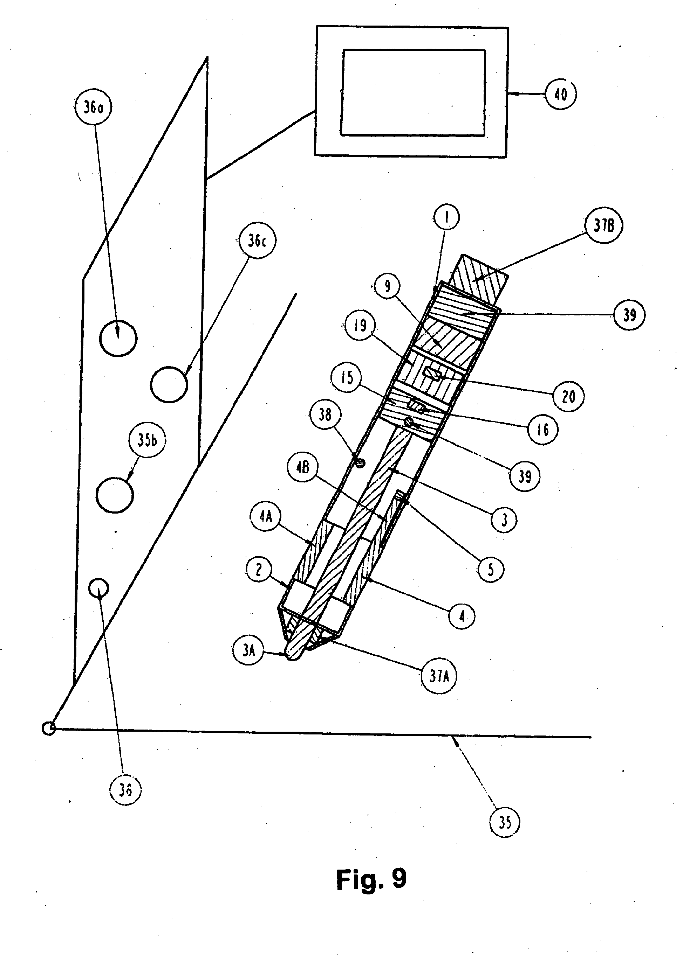

Figur 9 zeigt eine weitere Ausführungsform des erfindungsgemäßen Stiftes 1 zur Erfassung neuromotorischer Daten, wobei es sich um ein multisensorielles Schreibgerät handelt. Das in Figur 9 dargestellte Eingabesystem umfasst neben dem erfindungsgemäßen Schreibgerät 1 eine Schreibunterlage 35 und eine Feststation 36. Bei der in Figur 9 dargestellten Ausführungsform wird der erfindungsgemäße fluidische Stift 1 um eine weitere Sensorik zur akustischen Abstandsmessung erweitert. Hierzu ist an der Schreibstiftspitze 3A und an dem distalen Ende 3B des Schreibgerätes 1 jeweils ein Ultraschall-Wandler 37A, 37B vorgesehen. Neben der Ultraschall-Mess-Sensorik enthält der multisensorielle Schreibstift 1 einen fluidmechanischen Umsetzer mit einer Fluidkammer 4, die einen ersten ringförmigen oder spiralförmigen Fluidkammer-abschnitt 4A und einen kanalförmigen zweiten Fluidkammer-abschnitt 4B umfasst. Am Ende des kanalförmigen Fluidkammerabschnitts 4B ist ein Drucksensor 5 angebracht. Ferner enthält das Schreibgerät 1 einen Infrarotsender oder -empfänger 38 und ein Mikrophon 39. An der Stiftmine 3 ist über eine Halterung 15 der Permanentmagnet 16 angebracht. In der fest mit dem Stiftgehäuse 2 verbundenen Halterung 19 ist ein Magnetfeldsensor 20 eingelassen. Im hinteren Bereich des Schreibstiftgehäuses 2 befindet sich die Datenverarbeitungseinheit 9 sowie eine Batterie 39 zur Energieversorgung.

Bei der in Figur 9 dargestellten bevorzugten Ausführungsform des erfindungsgemäßen Stiftes 1 weist dieser neben der fluidischen Sensorik eine elektroakustische Abstandssensorik auf, mit welcher der absolute Ort der Stiftspitze 3A und die Neigung des Schreibstiftes 1 mittels akustischer Abtastmesstechnik bestimmt wird. Bei dem neuartigen System zur Erfassung einer handgeführten Bewegung eines Stiftes, wie es in Figur 9 dargestellt ist, sind entlang der Längsachse des Schreibstiftes 1 mindestens zwei akustische Wandler 37A, 37B angeordnet. Bei den elektroakustischen Wandlern handelt es sich vorzugsweise um Ultraschallsender oder um Ultraschallempfänger, die rotationssymmetrisch entlang der Längsachse im vorderen und hinteren Bereich des Schreibstiftes 1 fixiert sind.

Darüber hinaus sind in der Feststation 36 mindestens drei ortsfeste akustische Referenzwandler 36A, 36B, 36C vorgesehen, deren absolute Positionskoordinaten bekannt sind. Bei den elektroakustischen Referenzwandlern 36A, 36B, 36C handelt es sich entweder um Ultraschall-Sender oder um Ultraschall-empfänger.

Vorzugsweise sind die Referenzwandler 36A, 36B, 36C Ultraschallsender und die akustischen Wandler 37A, 37B, die an dem Schreibgerät 1 angebracht sind, Ultraschallempfänger. Dies hat den Vorteil, dass der Energieverbrauch des Schreibgerätes 1 minimiert wird und die Abstandsmessungen ohne zusätzliche Schallreflektoren möglich sind. Die Ultraschallwandler 37A, 37B bzw. 36A, 36B, 36C bestehen vorzugsweise aus zylindrischen oder ringförmigen piezoelektrischen Polymer-Folien, die in allen Richtungen rotationssymmetrisch bei festen Frequenzen, beispielsweise bei 40 bis 80 KHz Ultraschallpulse senden oder empfangen können. Die externe Station 36 enthält zusätzlich einen Infrarot-Empfänger bzw. -Sender 36D. Ein zwischen den Infrarot-Sendern bzw. -Empfängern 36D, 38 übertragener Infrarot-Impuls startet die Ultraschall-Laufzeitmessung der Ultraschallpulse zwischen den Ultraschallempfängern und den Ultraschallsendern. Dabei werden bei bekannter Schallgeschwindigkeit VS die verschiedenen Abstände zwischen den beiden Ultraschallwandlern 37A, 37B und den ortsfesten Ultraschallwandlern 36A, 36B, 36C berechnet. Aus den Abstandsdaten lassen sich eindeutig die Position der Schreibstiftspitze 3A und die Neigung des Schreibstiftes 1 im Zeittakt des Infrarot-Senders berechnen. Aus dem zeitlichen Verlauf der Positions- und Neigungswerte werden die Geschwindigkeiten und Beschleunigungen berechnet. Die Daten der so gewonnenen 3D-Kinematik werden anschließend der Merkmals-Extraktion zur Verfügung gestellt. Diese Abstandsmesstechnik wird auch als SAPS-Messtechnik (Ultra Sonic Absolute Position Sensing) bezeichnet.

Das Eingabesystem enthält optional zusätzlich eine mit einer Feststation 36 verbundenen Anzeigevorrichtung 40. Die ermittelten Positionswerte der Stiftspitze geben unabhängig von der Neigung des Schreibgeräts 1 die Position im Raum und das Tintenschriftbild oder die Zeichnung auf der Schreibunterlage 35 wieder.

Das in Figur 9 dargestellte neuartige System zur Erfassung einer handgeführten Bewegung des Stiftes umfasst mindestens zwei akustische Wandler 37A, 37B, die voneinander entlang einer Längsachse des Stiftes bzw. Schreibgerätes 1 angeordnet sind, mindestens drei ortsfeste akustische Referenzwandler 36A, 36B, 36C, die vorgegebene Koordinaten aufweisen, wobei mittels Schallsignallaufzeiterfassung die Abstände der an dem beweglichen Stift angeordneten akustischen Wandler 37A, 37B von den ortsfesten akustischen Referenzwandlern 36A, 36B, 36C ermittelt werden und auf der Grundlage der ermittelten Abstände die Position und die Neigung des Stiftes berechnet wird.

Die Kinematik der Schreib- und Zeichenbewegungen wird über den zeitlichen Ortsverlauf der Schreibstiftspitze 3A und die Neigung wird über die Anstellwinkel *α*, *β* des Schreibstiftes 1 in Längsachsenrichtung gemessen. Die Schreibdynamik wird über den Minenschreibdruck, d. h. den Druck der Schreibunterlage 35 auf den Schreibstift 3, in axialer Richtung und über den Fingerschreibdruck an der äußeren Hülle der Fluidkammer 4 des Schreibgeräts 1 erfasst. Position und Neigung werden mit der in Figur 9 dargestellten Ultraschall-Abstandssensorik erfasst. Der Schreibdruck auf die Schreibstiftmine 3 wird mit Magnetsensorik ermittelt. Ferner wird der Fingergreifdruck über fluidische Drucktechnik gemessen. Die durch das Schreiben hervorgerufenen Schwingungen des Stiftkörpers werden über das eingebaute Mikrophon 39 oder über eine Piezo-Folie erfasst. Das multisensorielle Schreibgerät 1, wie es in Figur 1 dargestellt ist, ist mittels einer Datenübertragungsleitung oder drahtlos mit einem Computer verbunden. Bewegte Objekte auf dem Display 14 kann die Testperson mit der Hand nachfahren. Das Mikrophonsignal liefert optional zusätzlich medizinische und biometrische Daten für die Handschrift und die Erkennung der Stimme.

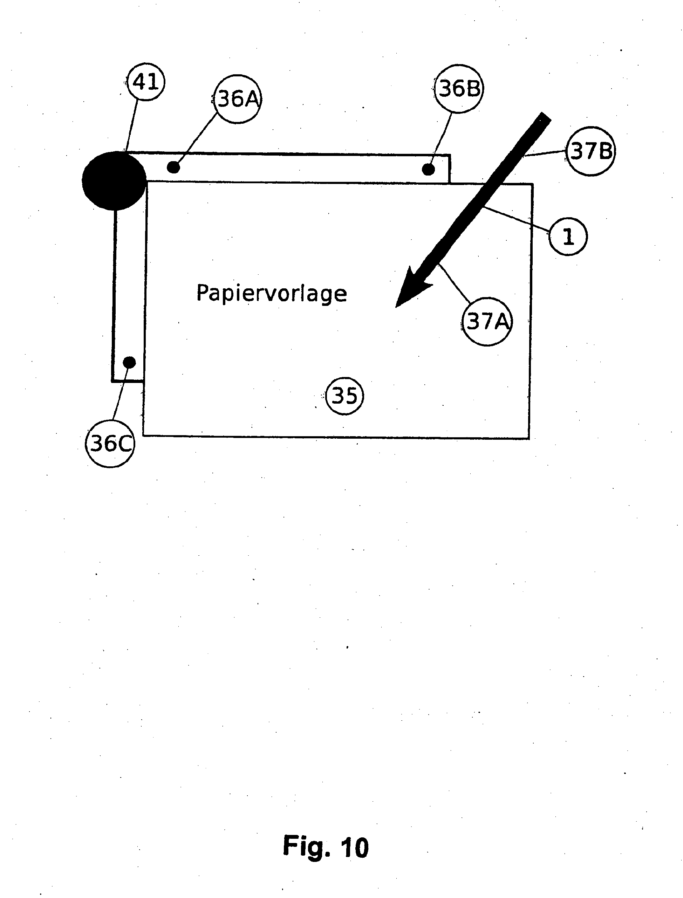

Es sind drei Ultraschallwandler 36A, 36B, 36 mit einer Docking-Station 41 vorgegeben. Die in Figur 10 dargestellte Anordnung stellt eine einfache aufklappbare Empfangeinheit dar, die an die Papiervorlage bzw. an eine Schreibunterlage 35 gelegt wird.

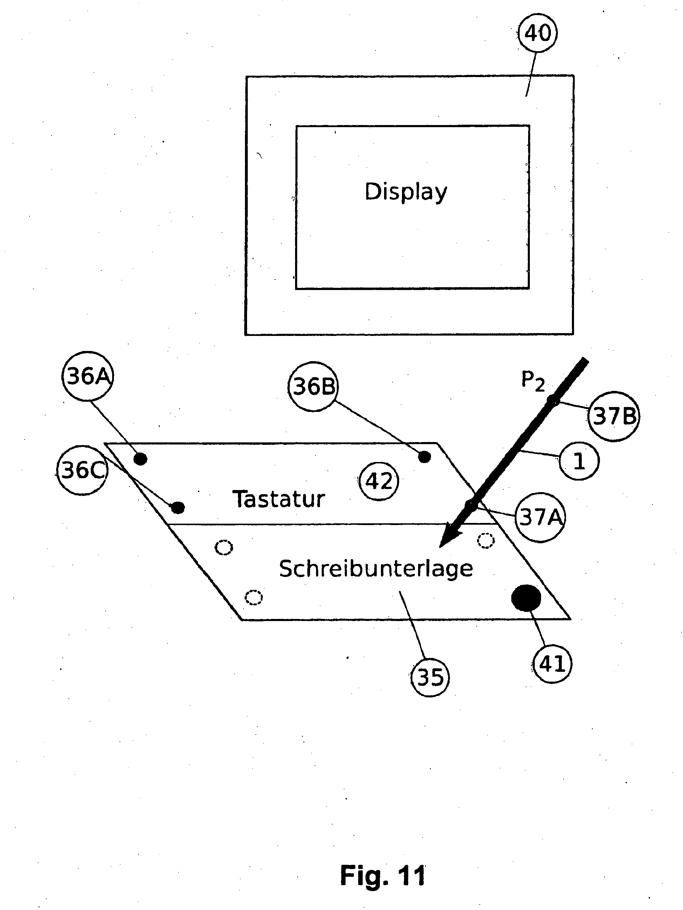

Figur 11 zeigt das erfindungsgemäße Schreibgerät 1 zusammen mit einem externen Smart Display 40. Drei externe Ultraschallwandler 36A, 36B, 36C sind in einem externen Modul fest positioniert, das eine Tastatur 42 und eine Schreibunterlage 35 umfasst. Das Schreibgerät 1 umfasst zwei Ultraschallwandler 37A, 37B. An dem Modul ist eine Docking-Station 41 vorgesehen.

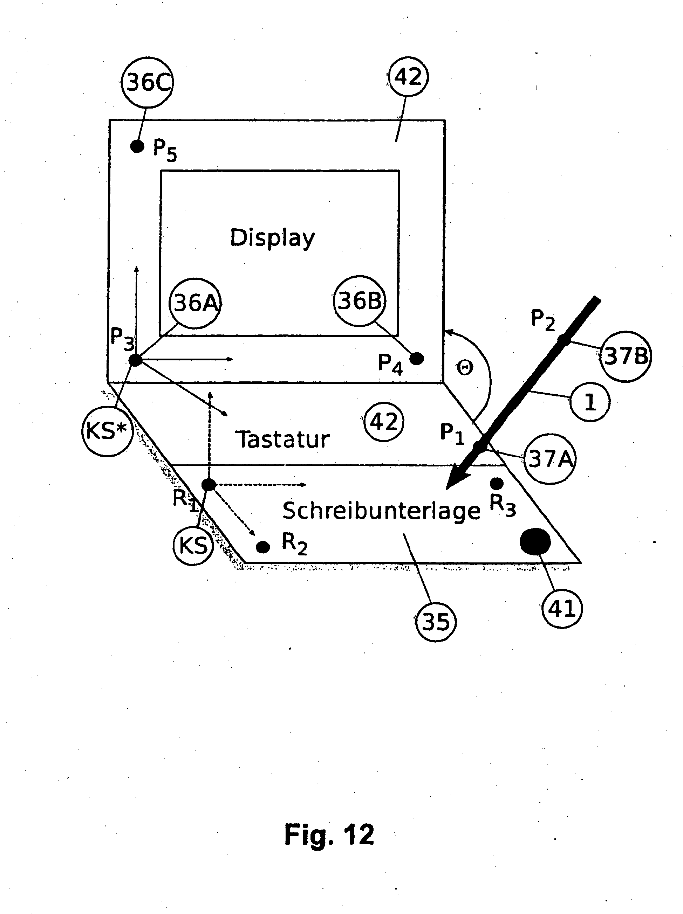

Figur 12 zeigt das erfindungsgemäße Schreibgerät 1 in Verbindung mit einem Notebook. Die Schreibunterlage 35 weist drei gesetzte Referenzpunkte R1, R2, R3 auf. Drei festpositionierte Ultraschallwandler 36A, 36B, 36C an den Positionen P3, P4, P5 sind im Display-Rahmen oder alternativ im Tastaturrahmen des Notebooks 1 vorgesehen. Wie in Figur 12 dargestellt ist, wird das Notebook unter einem Winkel ┌ aufgeklappt.

Zur Erfassung der Kinematik und der Dynamik der handgeführten Bewegungen des Schreibstiftes 1 auf der Schreibunterlage 35 oder im Raum über der Unterlage 35 ist, wie in Figur 12 dargestellt, das Schreibgerät 1 in einem Notebook integriert. Zunächst wird der Notebook-Deckel 43 unter einem frei wählbaren Winkel r aufgeklappt. Das kartesische Koordinatensystem des Notebooks wird als Laborsystem KS* automatisch aus den festen Positionen der drei Ultraschallwandler 36A, 36B, 36C und dem Winkel ┌ festgelegt. Auf die im Notebook integrierte Schreibunterlage 35 wird vorzugsweise Schreibpapier gelegt.

Das Schreibgerät 1 wird von dem Benutzer aus der Docking-Station 41 entnommen. Dadurch wird automatisch das Schreibgerät 1 und die im Notebook gespeicherte Erfassungssoftware aktiviert.

Anschließend wird der Schreibstift 1 auf der Schreibunterlage 35 bzw. dem darauf gelegten Papier aufgesetzt und die Positionsdaten für drei frei wählbare vorgegebene Referenzpunkte R1, R2, R3 aufgenommen. Dadurch wird automatisch das Koordinatensystem KS für die Papierunterlage 35 als Papiersystem definiert.

Anschließend werden handgeführte Bewegungen durch die Bedienperson bzw. Testperson mit dem Schreibgerät 1 auf der Schreibunterlage 35 oder im freien Raum über der Unterlage 35 durchgeführt und daraus die 3D-Kinematik und die Druckdynamik aus den Zeitreihen der Sensorsignale des Schreibgeräts 1 bestimmt.

Die kinematisch und dynamischen Daten werden zur anwendungsorientierten Verarbeitung zwischengespeichert und nach Beendigung der handgeführten Bewegungen wird das Schreibgerät 1 in die Docking-Station 41 zurückgesetzt.

Abschließend erfolgt optional ein Datentransfer von dem Schreibgerät 1 zu dem Notebook und das Schreibgerät 1 wird abgeschaltet.

Mittels der im Notebook abgespeicherten Software erfolgt eine anwendungsorientierte Merkmals-Extraktion, eine Merkmalsgewichtung und -klassifikation zur Herausgabe eines Diagnoseergebnisses als audiovisuelles Feedback. Das erfindungsgemäße Erfassungssystem erlaubt die gleichzeitige Erfassung biometrischer neuromotorischer Daten mit dem gleichen Eingabesystem. Das erfindungsgemäße Schreibgerät 1 eignet sich zur computergestützten Diagnose und Therapie neuromotorischer Krankheiten sowie zur quantitativen Analyse der neuromotorischen Auswirkung von Medikamenten, Drogen oder Stress. Darüber hinaus eignet sich das erfindungsgemäße Schreibstift-System zur computerunterstützten Diagnose und Therapie von Personen mit Schreibproblemen oder Reaktions- und Konzentrationsproblemen. Ferner kann eine Computereingabe von handgeschriebenen oder handgezeichneten Objekten erfolgen. Mit dem erfindungsgemäßen Schreibgerät 1 ist es ferner möglich, die Gestik von Fingern, Hand und Arm zu erfassen oder eine biometrische Online-Handschriftenauthentifizierung einer Person vorzunehmen. Das erfindungsgemäße Schreibgerät 1 lässt sich mit handelsüblichen Computern, wie PC, Notebook, Tablett oder mit einem Smart Display elektrisch verbinden. Insbesondere für die Telemedizin oder die Überwachung von Patenten kann das erfindungsgemäße Schreibgerät 1 zusammen mit einem Pocket PC, PDA, Handy oder als mobiles Diagnosesystem ausgelegt werden.

Das erfindungsgemäße Schreibgerät 1 hat den Vorteil, dass es bei der Nutzung eine hohe Akzeptanz aufgrund des vertrauten Umgangs mit einem Schreibstift aufweist. Darüber hinaus ist das erfindungsgemäße Schreibgerät 1 technologisch leicht realisierbar und weist eine relativ geringe Komplexität auf. Zusätzlich kann das erfindungsgemäße Schreibgerät 1 neben der Ermittlung neuromotorischer Daten im Bereich der Biometrie und von Mensch-Maschine-Interfaces eingesetzt werden. Somit kann das erfindungsgemäße Eingabesystem insbesondere folgende Störungen bzw. Krankheiten untersuchen, nämlich Tremorformen, Parkinson, Dystonie, Epilepsie, Schlaganfall, unfallbedingte Bewegungsstörungen, neuropathologische Erscheinungen, Drogenkonsum sowie motorische Schreibprobleme.

### Bezugszeichenliste

- 1: Schreibgerät
- 2: Schreibgerätgehäuse
- 3: Stiftmine
- 4: Fluidkammer
- 5: Drucksensor
- 6: Finger
- 7: Piezo-Folie
- 8: Hülse
- 9: Datenverarbeitungseinheit
- 10: Fluid
- 11: Gas
- 12: Elektrode
- 13: Elektrode.
- 14: Spule
- 15: Halterung
- 16: Permanentmagnet
- 17: Mikrophon
- 18: Gummiring
- 19: Halterung
- 20: Magnetfeldsensor
- 21: Eisenring
- 22: Minenlagerung
- 23: Magnetschicht
- 24: Piezo-Folie
- 25: Magnetfeldsensor
- 26: Magnetfeldsensor
- 27: Magnet
- 29: Magnet
- 30: Feder
- 32: Halterung
- 33: Teflonkugel
- 34: Ring
- 35: Schreibunterlage
- 36: Feststation
- 37: Ultraschallwandler
- 38: Infrarot-Sender
- 39: Mikrophon
- 40: Display
- 41: Docking-Station
- 42: Tastatur
- 43: Notebook-Display

## Patentansprüche

1. Fluidischer Stift (1) zur Erfassung neuromotorischer Daten bei einer handgeführten Bewegung des fluidischen Stiftes (1),
wobei der fluidische Stift (1) aufweist:
(a) mindestens eine Fluidkammer (4), die eine geschlossene elastische Wandung aufweist und mit einem Fluid (10) gefüllt ist, wobei der Flüssigkeitsdruck (P) innerhalb der Fluidkammer (4) einer bei der handgeführten Bewegung des fluidischen Stiftes (1) auftretenden Fingerdruckkraft von Greiffingern (6) und der bei einer Beschleunigung auftretenden Trägheitskräfte entspricht, welche auf einen ersten Fluidkammerabschnitt (4A) der Fluidkammer (4) drücken, und
(b) mindestens einen Drucksensor (5), der den Flüssigkeitsdruck (P) innerhalb der Fluidkammer (4) erfasst und in ein elektrisches Signal für eine Datenverarbeitungseinheit (9) umwandelt.

2. Fluidischer Stift nach Anspruch 1,
**dadurch gekennzeichnet,**
**dass** der erste Fluidkammerabschnitt (4A) der Fluidkammer (4) spiral- oder ringförmig ist.

3. Fluidischer Stift nach Anspruch 1 oder 2,
**dadurch gekennzeichnet,**
**dass** die Fluidkammer (4) einen zweiten Fluidkammerabschnitt (4B) aufweist, der kanalförmig ist und in Längsrichtung des Stiftes (1) angeordnet ist.

4. Fluidischer Stift nach Anspruch 2,
**dadurch gekennzeichnet,**
**dass** der mindestens eine Drucksensor (5) an dem ersten Fluidkammerabschnitt (4A) der Fluidkammer (4) vorgesehen ist.

5. Fluidischer Stift nach Anspruch 1 bis 4,
**dadurch gekennzeichnet,**
**dass** das Fluid (10) eine elektrisch leitende Flüssigkeit ist.

6. Fluidischer Stift nach Anspruch 1 bis 4,
**dadurch gekennzeichnet,**
**dass** das Fluid (10) eine elektrisch nicht leitende Flüssigkeit ist.

7. Fluidischer Stift nach Anspruch 1 bis 4,
**dadurch gekennzeichnet,**
**dass** das Fluid (10) eine magneto-rheologische Flüssigkeit ist.

8. Fluidischer Stift nach Anspruch 1 bis 7,
**dadurch gekennzeichnet,**
**dass** der Drucksensor (5) den Flüssigkeitsdruck kapazitiv, elektroresistiv oder induktiv erfasst.

9. Fluidischer Stift nach Anspruch 1,
**dadurch gekennzeichnet,**
**dass** die elastische Wandung der Fluidkammer (4) aus einem elastischen Schlauch besteht.

10. Fluidischer Stift nach Anspruch 2,
**dadurch gekennzeichnet,**
**dass** der ringförmige erste Fluidkammerabschnitt (4A) durch eine ringförmige Nut in einem Stiftkörper (2) des Stifts (1) gebildet ist, die mit einer elastischen Hülse (8) überzogen ist.

11. Fluidischer Stift nach Anspruch 10,
**dadurch gekennzeichnet,**
**dass** die elastische Hülse (8) auf einer Innen- oder Außenseite eine elastische Piezoschicht (8B) aufweist.

12. Fluidischer Stift nach Anspruch 11,
**dadurch gekennzeichnet,**
**dass** die elastische Hülse (8) den ringförmigen Fluidkammerabschnitt (4A) und zumindest teilweise den zylinderförmigen Stiftkörper (2) überzieht.

13. Fluidischer Stift nach Anspruch 2,
**dadurch gekennzeichnet,**
**dass** der spiralförmige erste Fluidkammerabschnitt (4A) mehrere Windungen aufweist, zwischen denen mindestens eine elastische Piezofolie (7) angeordnet ist.

14. Fluidischer Stift nach Anspruch 13,
**dadurch gekennzeichnet,**
**dass** die elastischen Piezofolien (7) streifenförmig sind und in Längsrichtung des Stiftes (1) angeordnet sind.

15. Fluidischer Stift nach Anspruch 14,
**dadurch gekennzeichnet,**
**dass** für jeden Greiffinger (6), der den fluidischen Stift (1) bei der handgeführten Bewegung greift, eine zugehörige Piezofolie (7) vorgesehen ist.

16. Fluidischer Stift nach Anspruch 1,
**dadurch gekennzeichnet,**
**dass** der fluidische Stift (1) eine Stiftmine (3) aufweist.

17. Fluidischer Stift nach Anspruch 16,
**dadurch gekennzeichnet,**
**dass** die Stiftmine (3) eine Stiftspitze (3A) aufweist.

18. Fluidischer Stift nach Anspruch 17,
**dadurch gekennzeichnet,**
**dass** an der Stiftspitze (3A) und an einem gegenüberliegenden distalen Ende (3B) des Stiftes (1) jeweils ein Ultraschallwandler (37A, 37B) vorgesehen ist.

19. Fluidischer Stift nach Anspruch 18,
**dadurch gekennzeichnet,**
**dass** die Ultraschallwandler (37A, 37B) Ultraschallempfänger zum Empfang eines Ultraschallsignals sind.

20. Fluidischer Stift nach Anspruch 19,
**dadurch gekennzeichnet,**
**dass** die Ultraschallwandler (37A, 37B) Ultraschallsender zum Senden eines Ultraschallsignals sind.

21. Fluidischer Stift nach Anspruch 17,
**dadurch gekennzeichnet,**
**dass** der fluidische Stift (1) Magnetfeldsensoren zur Erfassung eines auf die Stiftspitze (3A) der Stiftmine (3)aufgebrachten Schreibdruckes aufweist.

22. Fluidischer Stift nach Anspruch 21,
**dadurch gekennzeichnet,**
**dass** die Magnetfeldsensoren GMR-Sensoren sind.

23. Fluidischer Stift nach Anspruch 21,
**dadurch gekennzeichnet,**
**dass** die Magnetfeldsensoren Hall-Sensoren sind.

24. Fluidischer Stift nach Anspruch 21,
**dadurch gekennzeichnet,**
**dass** an dem der Stiftmine (3) entgegengesetzten distalen Ende ein Permanentmagnet (16) vorgesehen ist.

25. Fluidischer Stift nach Anspruch 24,
**dadurch gekennzeichnet,**
**dass** der Magnetfeldsensor (20) zwischen dem Permanentmagneten (16) und einem zylinderförmigen ferromagnetischen Körper (21) angeordnet ist.

## Claims

1. Fluid pen (1) for detecting neuromotor data during a hand-guided movement of the fluid pen (1),
wherein the fluid pen (1) comprises:
a) at least one fluid chamber (4), which comprises a closed resilient wall and is filled with a fluid (10), the fluid pressure (P) inside the fluid chamber (4) corresponding to a finger compressive force of gripping fingers (6) during the hand-guided movement of the fluid pen (1) and to the inertial forces occurring during an acceleration, which press on a first fluid chamber portion (4A) of the fluid chamber (4),
b) at least one pressure sensor (5), which detects the fluid pressure (P) inside the fluid chamber (4) and converts it into an electric signal for a data processing unit (9).

2. Fluid pen according to claim 1, **characterised in that** the first fluid chamber portion (4A) of the fluid chamber (4) is spiral-shaped or annular.

3. Fluid pen according to either claim 1 or claim 2, **characterised in that** the fluid chamber (4) comprises a second fluid chamber portion (4B) which is duct-shaped and is arranged in the longitudinal direction of the pen (1).

4. Fluid pen according to claim 2, **characterised in that** the at least one pressure sensor (5) is provided on the first fluid chamber portion (4A) of the fluid chamber (4).

5. Fluid pen according to claim 1 to 4, **characterised in that** the fluid (10) is an electrically conductive fluid.

6. Fluid pen according to claim 1 to 4, **characterised in that** the fluid (10) is an electrically non-conductive fluid.

7. Fluid pen according to claim 1 to 4, **characterised in that** the fluid (10) is a magnetorheological fluid.

8. Fluid pen according to claim 1 to 7, **characterised in that** the pressure sensor (5) detects the fluid pressure in a capacitive, electro-resistive or inductive manner.

9. Fluid pen according to claim 1, **characterised in that** the resilient wall of the fluid chamber (4) consists of a resilient tube.

10. Fluid pen according to claim 2, **characterised in that** the annular first fluid chamber portion (4A) is formed by an annular groove, which is covered with a resilient sleeve (8), in a pen body (2) of the pen (1).

11. Fluid pen according to claim 10, **characterised in that** the resilient sleeve (8) comprises a resilient piezo layer (8B) on an inner or outer side.

12. Fluid pen according to claim 11, **characterised in that** the resilient sleeve (8) covers the annular fluid chamber portion (4A) and covers the cylindrical pen body (2) at least in part.

13. Fluid pen according to claim 2, **characterised in that** the spiral-shaped first fluid portion (4A) comprises a plurality of windings, between which at least one resilient piezo film (7) is arranged.

14. Fluid pen according to claim 13, **characterised in that** the resilient piezo films (7) are strip-shaped and are arranged in the longitudinal direction of the pen (1).

15. Fluid pen according to claim 14, **characterised in that**, for each gripping finger (6) which grips the fluid pen (1) during the hand-guided movement, an associated piezo film (7) is provided.

16. Fluid pen according to claim 1, **characterised in that** the fluid pen (1) comprises a pen refill.

17. Fluid pen according to claim 16, **characterised in that** the pen refill (3) comprises a pen tip (3A).

18. Fluid pen according to claim 17, **characterised in that** an ultrasound converter (37A, 37B) is provided in each case on the pen tip (3A) and on an opposite distal end (3B) of the pen (1).

19. Fluid pen according to claim 18, **characterised in that** the ultrasound converters (37A, 37B) are ultrasound receivers for receiving an ultrasound signal.

20. Fluid pen according to claim 19, **characterised in that** the ultrasound converters (37A, 37B) are ultrasound transmitters for transmitting an ultrasound signal.

21. Fluid pen according to claim 17, **characterised in that** the fluid pen (1) comprises magnetic field sensors for detecting a writing pressure applied to the pen tip (3A) of the pen refill (3).

22. Fluid pen according to claim 21, **characterised in that** the magnetic field sensors are GMR sensors.

23. Fluid pen according to claim 21, **characterised in that** the magnetic field sensors are Hall sensors.

24. Fluid pen according to claim 21, **characterised in that** a permanent magnet (16) is provided on the distal end opposite the pen refill (3).

25. Fluid pen according to claim 24, **characterised in that** the magnetic field sensor (20) is arranged between the permanent magnet (16) and a cylindrical ferromagnetic body (21).

## Revendications

1. Crayon fluidique (1) destiné à la détection de données neuromotrices grâce à un mouvement guidé par la main du crayon fluidique (1), le crayon fluidique (1) présentant :
(a) au moins une chambre à fluide (4), qui présente une paroi élastique fermée et est remplie d'un fluide (10), la pression de liquide (P) à l'intérieur de la chambre à fluide (4) correspondant à une force de pression du doigt de doigts de préhension (6) apparaissant lors du mouvement guidé par la main du crayon fluidique (1) et aux forces d'inertie apparaissant lors d'une accélération, qui font pression sur une première section de chambre à fluide (4A) de la chambre à fluide (4), et
(b) au moins un détecteur de pression (5), qui détecte la pression de liquide (P) à l'intérieur de la chambre à fluide (4) et convertit un signal électrique pour une unité de traitement de données (9).

2. Crayon fluidique selon la revendication 1, **caractérisé par le fait que** la première section de chambre à fluide (4A) de la chambre à fluide (4) présente une forme de spirale ou d'anneau.

3. Crayon fluidique selon la revendication 1 ou 2, **caractérisé par le fait que** la chambre à fluide (4) présente une seconde section de chambre à fluide (4B), qui a la forme d'un canal et est disposée dans la direction longitudinale du crayon (1).

4. Crayon fluidique selon la revendication 2, **caractérisé par le fait que** l'au moins un détecteur de pression (5) est prévu sur la première section de chambre à fluide (4A) de la chambre à fluide (4).

5. Crayon fluidique selon la revendication 1 à 4, **caractérisé par le fait que** le fluide (10) est un liquide électriquement conducteur.

6. Crayon fluidique selon la revendication 1 à 4, **caractérisé par le fait que** le fluide (10) est un liquide électriquement non conducteur.

7. Crayon fluidique selon la revendication 1 à 4, **caractérisé par le fait que** le fluide (10) est un liquide magnéto-rhéologique.

8. Crayon fluidique selon la revendication 1 à 7, **caractérisé par le fait que** le détecteur de pression (5) détecte la pression du liquide de manière capacitive, électro-résistante ou inductive.

9. Crayon fluidique selon la revendication 1, **caractérisé par le fait que** la paroi élastique de la chambre à fluide (4) est constituée d'un tuyau élastique.

10. Crayon fluidique selon la revendication 2, **caractérisé par le fait que** la première section annulaire de chambre à fluide (4A) est formée par une rainure annulaire dans un corps de crayon (2) du crayon (1), qui est recouverte d'un manchon élastique (8).

11. Crayon fluidique selon la revendication 10, **caractérisé par le fait que** le manchon élastique (8) présente sur une face intérieure ou extérieure un film piézoélectrique (8B) .

12. Crayon fluidique selon la revendication 11, **caractérisé par le fait que** le manchon élastique (8) recouvre la section de chambre à fluide (4A) de forme annulaire et au moins partiellement le corps de crayon de forme cylindrique (2).

13. Crayon fluidique selon la revendication 2, **caractérisé par le fait que** la première section de chambre à fluide (4A) en spirale présente plusieurs spires, entre lesquelles est disposé au moins un film piézoélectrique élastique (7).

14. Crayon fluidique selon la revendication 13, **caractérisé par le fait que** les films piézoélectriques élastiques (7) ont une forme de bande et sont disposés dans la direction longitudinale du crayon (1).

15. Crayon fluidique selon la revendication 14, **caractérisé par le fait qu'**un film piézoélectrique (7) respectif est prévu pour chaque doigt de préhension (6), qui saisit le crayon fluidique (1) grâce à un mouvement guidé par la main du crayon fluidique.

16. Crayon fluidique selon la revendication 1, **caractérisé par le fait que** le crayon fluidique (1) présente une mine de crayon (3).

17. Crayon fluidique selon la revendication 16, **caractérisé par le fait que** la mine de crayon (3) présente une pointe de crayon (3A).

18. Crayon fluidique selon la revendication 17, **caractérisé par le fait qu'**un transducteur ultrasonique (37A, 37B) est prévu respectivement sur la pointe de crayon (3A) et sur une extrémité distale opposée (3B) du crayon (1).

19. Crayon fluidique selon la revendication 18, **caractérisé par le fait que** les transducteurs ultrasoniques (37A, 37B) sont des récepteurs d'ultrasons destinés à la réception d'un signal ultrasonore.

20. Crayon fluidique selon la revendication 19, **caractérisé par le fait que** les transducteurs ultrasoniques (37A, 37B) sont des émetteurs d'ultrasons destinés à l'émission d'un signal ultrasonore.

21. Crayon fluidique selon la revendication 17, **caractérisé par le fait que** le crayon fluidique (1) présente des capteurs de champ magnétique pour détecter une pression d'écriture appliquée sur la pointe de crayon (3A) de la mine de crayon (3).

22. Crayon fluidique selon la revendication 21, **caractérisé par le fait que** les capteurs de champ magnétique sont des capteurs GMR.

23. Crayon fluidique selon la revendication 21, **caractérisé par le fait que** les capteurs de champ magnétique sont des capteurs à effet Hall.

24. Crayon fluidique selon la revendication 21, **caractérisé par le fait qu'**un aimant permanent (16) est prévu sur l'extrémité distale opposée à la mine de crayon (3).

25. Crayon fluidique selon la revendication 24, **caractérisé par le fait que** le capteur de champ magnétique (20) est disposé entre l'aimant permanent (16) et un corps ferromagnétique de forme cylindrique (21).
